**Europäisches Patentamt**

**European Patent Office**

**Office européen des brevets**

(11) Publication number : **0 529 751 A1**

# (12) EUROPEAN PATENT APPLICATION

(21) Application number : **92250209.1**

(22) Date of filing : **10.08.92**

(51) Int. Cl.⁵ : **C12N 5/00**

(30) Priority : **09.08.91 JP 224788/91**
**09.08.91 JP 200678/91**
**13.08.91 JP 203008/91**

(43) Date of publication of application :
**03.03.93 Bulletin 93/09**

(84) Designated Contracting States :
**DE FR GB IT**

(71) Applicant : **W.R. GRACE & CO.-CONN.**
**1114 Avenue of the Americas, Grace Plaza**
**New York, New York 10036-7794 (US)**

(72) Inventor : **Yamazaki, Manabu**
**5-37-405, 20 chome Tsurumakikita**
**Hdano-shi, Kanagawa-ken (JP)**
Inventor : **Mori, Yuichi**
**1642-212-B-4 Kamariva-cho, Kanazawa-ku**
**Yokohama-shi, Kanagawa-ken (JP)**
Inventor : **Tsuchida, Michiko**
**SAT III Atsugi 306, 671-1, Tomuro**
**Atsugi-shi, Kanagawa-ken (JP)**

(74) Representative : **UEXKÜLL & STOLBERG**
**Patentanwälte**
**Beselerstrasse 4**
**W-2000 Hamburg 52 (DE)**

(54) **Cell culture substrate, test material for cell culture and preparations thereof.**

(57) This invention provides cell culture substrates, cell culture test materials and preparation thereof using temperature-responsive polymeric compounds having an LCST lower than the cell culture temperature and cell adhesive substances capable of adhering the cell to be cultured without any deterioration of the cell and the cell adhesive substances having crosslinked parts and non-crosslinked parts in an island-in-sea pattern, a sea-sea pattern, a lamellar pattern or any mixture of these three patterns.

FIG. 1-(a)

FIG. 1-(b)

EP 0 529 751 A1

This application is a continuation-in-part of U.S. Patent Application Serial No. 675,375 filed March 26, 1991 which is a continuation-in-part of U.S. Patent Application Serial No. 492,691 filed March 13, 1990, abandoned.

Technical Field

The invention relates to a cell culture substrate and a method of preparing the same. Specifically, it relates to the use of a temperature- responsive polymeric compound and a cell adhesive substance capable of adhering a cell to be cultured for a cell culture substrate where subculture is required. The invention also relates to a cell culture test material using the cell culture substrate and a method of the preparation of the same. Specifically, it relates to a cell culture test material useful for determining optimal conditions for culturing a specific cell on the substrate.

Background Art of the Invention

The major applications of the current mammalian cell culture technology are (1) bioreactors for the production of cell products with physiological activities, (2) prosthesis for diseased or damaged living tissues or organs and (3) simulators to evaluate toxicity and activity of drugs and so on.

The mammalian cells used in cell culture technology can be divided into two types, i.e., anchorage independent cells and anchorage dependent cells.

Anchorage independent cells can perform their cellular functions such as survival, proliferation and production of substances without any substrate that serves as a scaffold for the cells. Typical examples are hybridomas formed from myeloma cells and spleen cells.

In contrast, the latter types of anchorage dependent cells are cells which normally cannot perform their cellular functions such as survival, proliferation and production of substances without a substrate that serves as a scaffold for the cells. The majority of normal diploid cells, including primary cells, are anchorage dependent. Even many of the established cell lines are known to show anchorage dependency. For example, the established cell lines for the production of useful cell products including cytokines, such as interferon and interleukin, differentiating factors such as erythropoietin, colony-stimulating factor, thrombopoietin, and tissue plasminogen activator and vaccines, are known to be anchorage dependent. In addition, most of the cells applicable as prostheses for a diseased or damaged living body and/or as simulators for evaluation of toxicity and activity of drugs, are intended to be anchorage dependent. Therefore, establishment of culturing technology for the anchorage dependent cells is extremely important for these applications.

In general, to utilize animal cells for these technological applications it is important to culture the cells in a large quantity and at a high density while keeping the cells at a full functional level. However, animal cells, more so than microbial cells, are highly susceptible to the effects of deficiency of supply of nutrients such as oxygen, and to the effects of accumulating of metabolic waste products.

In the case of anchorage independent cells, suspension culture techniques are considered to be the best. If cells are cultured in a suspension with agitation, waste materials can be removed quickly and nutrients can be supplied efficiently. Therefore, it is relatively easy to scale up the equipment for mass and high density culture for anchorage independent cells.

For anchorage dependent cells, it is not possible to use the suspension culture technique because the cells require a substrate for attachment. Therefore, different cell culture devices with a substrate for cell attachment have so far been developed. For example, on an experimental scale, dish-type, flask-type, and plate-type devices have been most widely used. However, the above mentioned devices are not suitable for mass cell culture.

Therefore, different ideas have been conceived to increase the surface area of the substrate where the cells could adhere, relative to the total volume. Examples of proposed devices include: (1) roller bottle type where bottles for cell culture are rotated to grow the cells on the entire surface of the wall, (2) multiple tray type where plates for cell adhesion are arranged parallel in the culture medium, and the culture medium is circulated among the plates, (3) coil type where a plastic film, formed into a coil, is inserted into a cylindrical tube which is rotated about its axis to adhere the cells, and then a culture medium is circulated among the film, (4) hollow fiber type where hollow fiber semipermeable membranes are contacted with the cells on the external surface of the hollow fibers and the culture medium is circulated through the interior of the hollow fiber membranes to supply nutrients and remove waste materials through the hollow fiber membranes, (5) packed glass bead type where cells are in contact with and adhered to the packed glass beads and the culture medium is circulated among them, and (6) microbead type where microbeads are suspended in the culture medium to attach the cells on the surface of the microbeads which are agitated to culture the cells.

As described above, attention has been mainly paid to morphological design of the cell culture device from the viewpoint of effectiveness in nutrient supply and waste removal. Recently, however, it has been found that it is almost impossible to maintain cell viability and functionality for a long period of time only by controlling the efficiency of nutrient supply and waste removal. Rather, it has been found that the cell culture substrate is a key to maintaining the cell viability and functionality for anchorage dependent cells. Therefore, research on the relationship between the nature of the cell culture substrate and cell functions has been actively carried out.

Heretofore, polystyrene has been most widely used as a material of cell culture substrates because of its optical transparency, non-toxicity, excellent mechanical properties, good moldability and low price. However, the cell adhesion process which leads to the cell proliferation process is significantly inhibited on the surface of the polystyrene culture substrate because of its hydrophobicity. Therefore, in order to improve cell attachment and proliferation, a modified hydrophilic polystyrene, endowed with negative charges by corona discharge treatment, has been developed and widely used as a cell culture substrate. However, it is found that the above described modification of polystyrene is still not enough for cells to express and maintain their specific functions.

Recently, studies have begun to bring the cell culture substrate closer to the *in vivo* environment in order to improve cell functions such as attachment, proliferation, differentiation, and production ability of cell products. Specifically, the studies incorporate substances capable of effectively controlling cell functions into the cell culture substrate. The most typical substance used to control the functions is an extracellular matrix component.

Study of the function of extracellular matrix components *in vivo* has progressed rapidly in recent years. It has become clear that an extracellular matrix component plays, not only a simple passive role such as supporting the cells and fixing the cells as known in the past, but also has a role in actively controlling or regulating cell functioning. Although a number of the extracellular matrix components have been identified, the most important component is collagen.

More than nine different types of collagen have been discovered, each of which is synthesized by a certain definite cell and is located in a certain cell tissue whereby the collagen plays a role of controlling different cell functions. Even with the same type of collagen, modification by introducing a variety of functional groups or modification of higher order structure can cause different effects on the cell functions. In addition to collagen, other substances identified as extracellular matrix components are: fibronectin, laminin, thrombospondin, vitronectin, proteoglycan and glycosaminoglycan. These substances have specific binding sites relative to the collagen and cell membrane and also play an important role in the cell attachment, proliferation and cell functions.

Furthermore, in addition to the above described extracellular matrix components, there are some other substances capable of effectively controlling cell functions such as attachment, proliferation and differentiation. Such substances include gelatin (a thermally denatured collagen), lectins which bind specifically to sugar moiety on the cell membrane, anchorage oligopeptides which are the binding sites of anchorage proteins such as fibronectin, and adhesive proteins isolated from shellfish.

As examples of the culture substrates combined with these substances which control the cell functions, collagen-coated substrate (K. Yoshizato, et al., Annals of Plastic Surgery, 13, 9, 1984), fibronectin-coated substrate (F. Grinnell, Expl. Cell Res., 102, 51, 1976) and the substrate coated with an adhesive protein of a shellfish (P. T. Picciano, et al., In Vitro Cellular and Developmental Biology 22(3), 24A, 1986) have been developed, and significant improvements on cell attachment and proliferation have been found.

Furthermore, recently a culture substrate coated with polystyrene containing a galactose-derivative group as a side chain has been developed, and some improvements on the attachment and viability of hepatocytes have been recognized (T. Akaike, et al., Jpn. J. Artif. Organs, 17, 227, 1988). By using the modified cell culture substrates as described above, it has become possible to culture cells which have not been able to attach and proliferate on the conventional culture substrate materials such as glass or polystyrene.

However, despite these advancements in culture devices and substrates, the current cell culture technology still has the following crucial problems.

A distinct feature of anchorage dependent cell cultures is that the cells stop further proliferation if the cells proliferate and completely cover the surface of the substrate. This is called contact inhibition. When contact inhibition occurs, a passage process, (that is, the process to detach the cells from the old substrate and then to transfer the detached cells to a new substrate) is necessary in order to continue the proliferation. In the past, proteolytic enzymes such as trypsin and collagenase, and EDTA as a calcium chelator were most commonly used for the cell detachment process. However, a conventional cell detachment process, like trypsinization, not only causes significant damage to the cell functions, but also presents significant obstacles to the cell culturing process.

The problems associated with the conventional detachment process are as follows:

(1) Conventional detaching agents destroy not only the bonds between cells and the culture substrate, but also bonds between neighboring cells. Three types of intercellular bonds, that is, tight junction, gap junction, and desmosome are known. The tight junction plays the role of a barrier to the permeability of substances between the apical and basal sides. Through the gap junction, the exchange of substances and information is carried out between the neighboring cells. By the desmosome, the cell assembly is mechanically supported. The cell is not able to be alive and functional alone, but the intercellular bonds enable the cell to express and maintain specific functions (B. Alberts et al., "Molecular Biology of the Cell", 3rd edn., Garland Publishing Inc., New York & London, p. 673 1983). Accordingly, the conventional detaching agent causes crucial damage to the functions of the cultured cells by destroying completely the intercellular bonds formed in the culturing process.

(2) On the cell membrane, there are many receptors for signaling molecules such as hormones, local chemical mediators, and neurotransmitters. The target cell communicates with the secreting cell through the specific reaction between the receptor and the signaling molecule. It has been found that the conventional detaching agents destroy the receptors (e.g. C. Sung, et al., Biochem. Pharmacol., 38, 696, 1989). Accordingly, the cells treated with the conventional detaching agents cannot be controlled by the signaling molecules. This means that the cell loses its specific functions.

(3) As a nutrient, the common culture medium contains serum which contains potent trypsin inhibitors. Therefore, prior to trypsinization, the cells have to be washed thoroughly with a buffer solution in order to remove the trypsin inhibitors. This washing procedure not only complicates the operation, but also enhances the risk of contamination which is a lethal problem in cell culture technology.

Thus, even if the cells with specific functions can be cultured by use of a sophisticated culture substrate combined with an extracellular matrix and an effective culture device design for supply of nutrients and removal of waste, the recovery of the cultured cells by use of the conventional detaching agents markedly damages the cellular functions. Particularly, the deterioration of cell functions induced by conventional cell recovery significantly reduces the ability of production of cell products. Also, by the cell recovery method, the self-supported cell assembly for prosthesis cannot be obtained because the cell detaching agents, such as trypsin, completely break the cell assembly.

Furthermore, the cells treated with the current recovery process are useful as simulators for the evaluation of activity of the drugs, since the membrane-bound receptors responsive to the drugs are completely digested by trypsin.

In addition, the conventional recovery process brings a crucial shortcoming particularly to the mass cell culture technology. Excessively low initial cell concentration of anchorage dependent cells in a culture medium is said to retard the proliferation of cells and the ability to produce substances even if the cells adhere to the substrate. Particularly for primary cells or normal diploid cells which are difficult to harvest, cultivation in a large volume of culture media from the start will reduce the cell concentration excessively. In such cases, the concentration of the cells has to be increased by repeating the cell culture in steps using a culturing device having a smaller capacity. This means that in a mass cell culture process, a lot of repeated cell recovery processes are necessary. Accordingly, the effect of the conventional cell detachment procedure is much more crucial compared to a small quantity cell culture process.

In order to solve the above described problems, the inventors of the present invention have been engaged in the development of a cell culture technique using temperature-responsive polymeric compounds for cell culture substrate. It has been found that the cell detachability from the cell culture substrate minutely differs depending on the types of the cells employed.

Objects

It is an object of the invention to provide a cell culture substrate, suitable for cell subculture, which avoids the above described problems.

It is another object of the invention to provide a cell culture test material suitable for easily judging the cell attachability and growth and detachability for various types of cells including cells having poor cell detachability with respect to a cell culture substrate.

Definition

The term "LCST" is used herein to mean a lower critical solution temperature which is a transition temperature of a temperature-responsive polymeric compound between hydration and dehydration.

## SUMMARY OF THE INVENTION

A cell culture substrate in accordance with the invention comprises (a) a temperature-responsive polymeric compound having an LCST lower than the culture temperature and (b) a cell adhesive substance capable of adhering the cell to be cultured without any deterioration of the cell, said cell adhesive substance having crosslinked parts and non-crosslinked parts in at least one pattern selected from the group consisting of an island-in-sea pattern, a sea-sea pattern and a lamellar pattern. The islands, seas and lamellar layers may be of any suitable shape.

The present invention includes a method of preparing a cell culture substrate which comprises forming a coating of (a) a temperature-responsive polymeric compound having an LCST lower than the cell culture temperature and (b) a cell adhesive substance capable of adhering the cell to be cultured without any deterioration of the cell and introducing crosslinks into said cell adhesive substance in at least one pattern selected from the group consisting of an island-in-sea pattern, a sea-sea pattern and a lamellar pattern.

In another aspect, the invention encompasses a cell culture test material comprising an array of coated portions on a supporting material, each of the coated portions comprising (a) a temperature-responsive polymeric compound having an LCST lower than the cell culture temperature and (b) a cell adhesive substance capable of adhering the cell to be cultured without any deterioration of the cell, each of the coated portions having substantially the same area, said cell adhesive substance in each of the coated portions having crosslinked parts and non-crosslinked parts in at least one common pattern selected from the group consisting of an island-in-sea pattern, a sea-sea pattern and a lamellar pattern, and the crosslinked parts of each coated portion having a degree of crosslinking which differs from the degree of crosslinking in each of the other coated portions.

In a further aspect, the invention encompasses a cell culture test material which comprises an array of coated portions on a supporting material, each of the coated poritons comprising (a) temperature-responsive polymeric compound having an LCST lower than the cell culture temperature and (b) a cell adhesive substance capable of adhering the cell to be cultured without any deterioration of the cell, said cell adhesive substance in each of the coated portions having crosslinked parts and non-crosslinked parts in at least one common pattern selected from the group consisting of an island-in-sea pattern, a sea-sea pattern and a lamellar pattern, the crosslinked parts of each coated portion having the same degree of crosslinking, and said common pattern having a degree of regularity which differs from the regularity of the common pattern in each of the other coated portions.

In a still further aspect, the invention encompasses a method of preparing a cell culture test material comprising:

i. forming a coating on a supporting material, said coating comprising (a) a tempetarure-responsive polymeric compound having an LCST lower than the cell culture temperature and (b) a cell adhesive substance capable of adhering the cell to be cultured without any deterioration and;

ii. irradiating said coating with ultraviolet rays through a combination of a photo mask bearing at least one pattern selected from the group consisting of an island-in-sea pattern, a sea-sea pattern and a lamellar pattern and a photo mask having an array of areas and each area being substantially equal in size and each area allowing a different percent transmission of the ultraviolet rays.

In still another aspect, the invention encompasses a method of preparing a cell culture test material comprising:

i. forming a coating on a supporting material, said coating comprising (a) a temperature-responsive polymeric compound having an LCST lower than the cell culture temperature and (b) a cell adhesive substance capable of adhering the cell to be cultured without any deterioration; and

ii. irradiating the coating with ultraviolet rays through a photo mask having an array of areas, each area being substantially equal in size, each area bearing at least one pattern selected from the group consisting of an island-in-sea pattern, a sea-sea pattern and a lamellar pattern, and each area allowing a different percent transmission of the ultraviolet rays.

In a still further aspect, the invention encompasses a method of preparing a cell culture test material comprising:

i. forming a coating on a supporting material, said coating comprising (a) a temperature-responsive polymeric compound having an LCST lower than the cell culture temperature and (b) a cell adhesive substance capable of adhering the cell to be cultured without any deterioration; and

ii. irradiating said coating with ultraviolet rays through a photo mask having an array of areas, each area having substantially the same size, each area bearing at least one pattern selected from the group consisting of an island-in-sea pattern, a sea-sea pattern and a lamellar pattern, said pattern in each area having a differing regularity, and each area allowing the same percent transmission of the ultraviolet rays.

5

In a yet further aspect, the invention encompasses a method of preparing a cell culture test material comprising:

i. forming a coating on a supporting material, said coating comprising (a) a temperature-responsive polymeric compound having an LCST lower than the cell culture temperature and (b) a cell adhesive substance capable of adhering the cell to be cultured without any deterioration and;

ii. sequentially irradiating said coating with ultraviolet rays at the same exposure energy through a photo mask having an area bearing at least one pattern allowing the transmission of said ultraviolet rays, the pattern being selected from the group consisting of an island-in-sea pattern, a sea-sea pattern and a lamellar pattern, and the regularity of said pattern being different for each irradiation.

In another aspect, the invention embraces a method of preparing a cell culture test material comprising:

i. forming a coating on a supporting material, said coating comprising (a) a temperature-responsive polymeric compound having an LCST lower than the cell culture temperature and (b) a cell adhesive substance capable of adhering the cell to be cultured without any deterioration and;

ii. sequentially irradiating said coating with ultraviolet rays at different exposure energies through a photo mask having an area bearing at least one pattern allowing the transmission of said ultraviolet rays, said pattern being selected from the group consisting of an island-in-sea pattern, a sea-sea pattern and a lamellar pattern, the position of said area on said coating being different for each irradiation.

BRIEF DESCRIPTION OF THE DRAWINGS

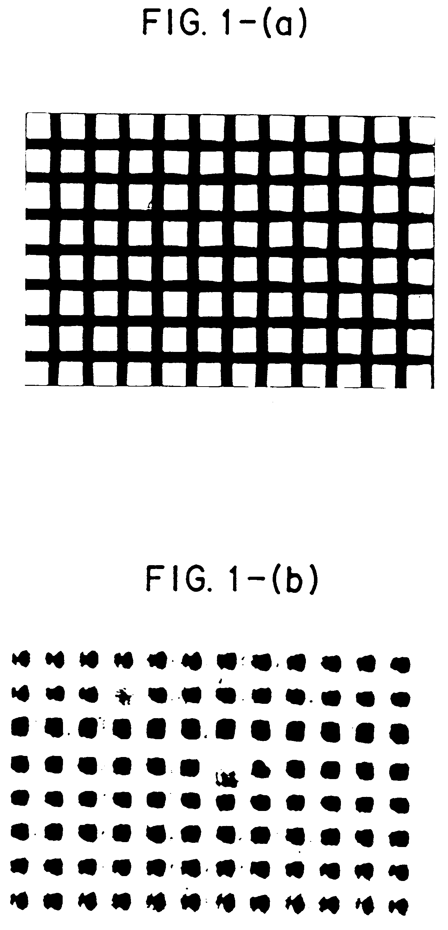

FIG. 1-(a) is a microscopic photograph, at 170 magnification, of a stainless steel mesh having a size of 500 meshes and a line width of 18 μm which was used in the preparation of one embodiment of the cell culture substrate in accordance with the present invention.

FIG. 1-(b) is a microscopic photograph, at 170 magnification, of the cell culture substrate prepared by using the stainless steel mesh of FIG. 1-(a). In the photograph, the black portions show crosslinked collagen.

FIG. 2-(a) and FIG. 2-(b) are schematic plan views of embodiments of a photo mask which can be employed in the preparation of the cell culture test material in accordance with the present invention.

FIG. 3-(a), FIG. 3-(b), and FIG. 3-(c) are schematic plan views of other embodiments of a photo mask which can be employed in the preparation of the cell culture test material in accordance with the present invention.

DETAILED DESCRIPTION OF THE INVENTION

The temperature-responsive polymeric compound having an LCST lower than the cell culture temperature, which can be used as one component of the cell culture substrate in this invention, is in a solid state at the cell culture temperature. If the temperature is lowered to a temperature below the LCST, the temperature-responsive polymeric compound becomes soluble in the culture medium, and the cells on the cell culture substrate will be detached therefrom.

Examples of the temperature-responsive polymeric compounds having an LCST lower than the cell culture temperature which can be used as one component of the cell culture substrate in the present invention are poly-N-substituted (meth)acrylamide derivatives and their copolymers, polymethylvinylether, polyethylene oxide, etherized methylcellulose, and partially acetylated polyvinyl alcohol. Of these preferred compounds, more preferred are poly-N-substituted acrylamide derivatives, poly-N-substituted methacrylamide derivatives and their copolymers.

Preferred examples of such temperature-responsive polymeric compounds in the present invention are listed below, but this invention is not limited to these examples. The LCST's of these polymers rise with the sequence of polymers listed below.

Poly-N-acryloyl piperidine, poly-N-n-propyl methacrylamide, poly-N-isopropyl acrylamide, poly-N,N-diethyl acrylamide, poly-N-isopropyl methacrylamide, poly-N-cyclopropyl acrylamide, poly-N-acryloylpyrrolidine, poly-N,N-ethylmethyl acrylamide, poly-N-cyclopropyl acrylamide, poly-N-ethyl acrylamide.

The above described polymers may be homopolymers or copolymers with other monomers. Any hydrophilic monomers or hydrophobic monomers can be used as the monomers for copolymerization. Generally speaking, the copolymerization with a hydrophilic monomer will raise the LCST, and the copolymerization with a hydrophobic monomer will lower the LCST. With a proper selection of monomers, a copolymer with a desired LCST can be achieved.

Examples of suitable hydrdophilic monomers are N-vinylpyrrolidone, vinylpyridine, acrylamide, methacrylamide, N-methylacrylamide, hydroxyethyl methacrylate, hydroxyethyl acrylate, hydroxymethyl methacrylate, hydroxymethyl acrylate, acrylic acid and methacrylic acid having acidic groups and its salts, vinylsulfonic acid, styrylsulfonic acid and N,N-dimethylaminoethyl methacrylate, N,N-diethylaminoethyl methacrylate, and N,N-dimethylaminopropyl acrylamide having a basic group and their salts, but the present invention is not limited to these compounds.

Examples of suitable hydrophobic monomers are acrylate derivatives and methacrylate derivatives such as ethyl acrylate, methyl methacrylate and glycidyl methacrylate; N-substituted alkyl (meth)acrylamide derivatives such as N-n-butyl (meth)acrylamide; vinyl chloride, acrylonitrile, styrene and vinyl acetate, but the present invention is not limited to these compounds.

The molecular weight of the temperature-responsive polymeric compound which can be employed in the present invention is preferably at least about $1.0 \times 10^5$, and more preferably higher than about $1.0 \times 10^6$. The molecular weight herein means a number average molecular weight obtained from the viscosity. For example, the relationship between the number average molecular weight ($\overline{Mn}$) of poly-N-isopropyl acrylamide and its intrinsic viscosity [η] can be represented by the follwing equation of [S. Ito and R. T. Geronimo, Sen'i Kobunshi Zairyo Kenkyusho Hokoku, No. 159, p. 23 (1988)];

$$[\eta] = 9.59 \times 10^{-5}\overline{Mn}^{0.65}$$

(in tetrahydrofuran solution at 27 ° C)

The cell adhesive substance capable of adhering a cell to be cultured without any deterioration of the cell which can be employed in the present invention is collagen or a mixture of collagen as a major component and at least one substance, as a minor component, selected from the group consisting of fibronectin, laminin, vitronectin, proteoglycan, glycosaminoglycan, thrombospondin, gelatin, lectins, anchorage oligopeptides and adhesive proteins isolated from shellfish. There is no restriction on the types of collagens which can be employed as the cell adhesive substance of the present invention.

It is known that, in general, collagen forms fibrils in the cell culture and takes the same structure as present in a living body, and the collagen in this state hardly dissolves in the culture medium. However, when collagen is present with a temperature-responsive polymeric compound, this fibril-forming ability of collagen cannot fully be exhibited. When the formation of fibrils is not sufficient, part of collagen dissolves in the culture medium before the cells adhere to the cell culture substrate. In order to cope with this dissolution of collagen, crosslinks are introduced into part of collagen. As a result, the collagen becomes insoluble in the culture medium and the cell can sufficiently adhered to the cell culture substrate.

On the other hand, cells can adhere on to the cell culture substrate at their binding sites which exist on the surface of the cell membrane in a mozaic pattern and can grow on the cell culture substrate. In this instance, the cells adhere onto the substrate at multiple sites. The increased numbers of such adhering sites increase the adhering strength of the cells to the substrate. Accordingly, it will become more difficult to detach the cells from the substrate. Thus, in culturing a cell having poor detachability from the cell culture substrate, it is advantageous to decrease the number of such adhering sites to some extent.

It has now been found that the use of a cell culture substrate where crosslinked parts and non-crosslinked parts in the cell adhesive substance are configured in an island-in-sea pattern, a sea-sea pattern or a lamellar pattern can regulate the number of the adhering sites to improve the cell detachability. However, the ease of cell culturing depends on not only such a configuration of the cell adhesive substance but also the degree of crosslinking in the crosslinked parts of the cell adhesive substance. It has also been found that, in general, increased degrees of crosslinking tend to increase the cell attachability and cell growth ability, but reduce the cell detachability, while decreased degrees of crosslinking tend to reduce the cell attachability and the cell growth ability but improve the cell detachability.

Thus, as a characteristic feature of the present invention, the cell culture substrate has a structure where crosslinked parts and non-crosslinked parts of the cell adhesive substance exist as a mixture in such a specified pattern that the cell adheres mainly on to the crosslinked parts.

The specified pattern of the crosslinked parts and the non-crosslinked parts in the cell adhesive substance in the present invention is preferably either an island-in-sea pattern, a sea-sea pattern or a lamellar pattern or any mixture of these three patterns. The islands, seas, and lamellar layers may be of any suitable shape. Further, these patterns may have the same or different regularity. The optimal pattern may vary depending on the types of the cells employed.

The term "regularity" in the patterns used herein means the distance between the centers of two adjacent islands in the island-in-sea pattern, the repeat period between two adjacent seas in the sea-sea pattern, or the repeat period between two adjacent layers in the lamellar pattern.

When the pattern is an island-in-sea pattern, the portions where crosslinks have been incorporated may be islands or sea. It is preferred that the size of the islands and the distance between two adjacent islands are not greater than the size of a cell to be cultured, respectively. The average size and the average distance are preferably not greater than 50 μm, respectively, and more preferably not greater than 20 μm, respectively. The minimal average size and distance are typically 0.1 μm, respectively.

When the pattern is a sea-sea pattern or a lamellar pattern, it is preferred that the period is not greater than two times the size of a cell to be cultivated. The term "repeat period" used herein means an average distance between adjacent pairs of parts, i.e., crosslinked parts and non-crosslinked parts when the cell culture substrate is cut by an arbitrary line. The repeat period is preferably not greater than 100 μm, and more preferably not greater than 40 μm.

Further, the total area of the crosslinked parts in the cell adhesive substance is preferably not greater than 50% of the cell growth area, and more preferably not greater than 30% of the cell growth area.

The cell culture substrate of the present invention typically consists of a coating of the temperature-responsive polymeric compound having an LCST lower than the cell culture temperature and the cell adhesive formed on a supporting material, and the cell adhesive substance having crosslinked parts and non-crosslinked parts in a specified pattern.

The cell culture substrate of the present invention can be prepared by coating an aqueous solution of the mixture of the temperature-responsive polymeric compound and the cell adhesive substance on the entire surface of the supporting material at a temperature below the LCST, drying the coating thus obtained, and introducing crosslinks into the cell adhesive substance in a specified pattern. The cell culture substrate of the present invention can also be prepared by dipping the supporting material into the aqueous mixture solution of the temperature-responsive polymeric compound and the cell adhesive substance at a temperature below the LCST, drying the coating thus obtained, and introducing crosslinks into the cell adhesive substance in a specified pattern.

The mixing weight ratio of the temperature-responsive polymeric compound to the cell adhesive substance which can be employed in the present invention is typically from about 49:1 to about 9:1, and preferably from about 39:1 to about 9:1. This ratio varies depending on the type of the employed cell adhesive substance within the above described range.

The thickness of the coating after it is dried is at least about 0.5 μm to about 100 μm, preferably about 1.0 μm to 10 μm. When the thickness is below about 0.5 μm, the cell detachability remarkably worsens, and it takes a very long time for the detachment of the cells resulting in the cell functions being rendered unstable. The thickness of the coating can be calculated from the concentration of the aqueous solution of the temperature-responsive polymeric compound and the cell adhesive substance and the thickness of the coating before drying, on condition that the density of the substances forming the coating is assumed to be 1.0.

The cell culture substrate of the present invention also can be prepared by forming a layer of the temperature-responsive polymeric compound on a supporting material, forming a layer of the cell adhesive substance on the layer of the temperature-responsive polymeric compound and introducing crosslinks into the layer of the cell adhesive substance in a specified pattern. Also, the cell culture substrate of the present invention can be prepared by forming a layer of the cell adhesive substance on a supporting material, forming a layer of the temperature-responsive polymeric compound on the layer of the cell adhesive substance, and introducing crosslinks into the layer of the cell adhesive substance in a specified pattern.

The method of introducing crosslinks into the cell adhesive substance in a specified pattern which can be employed in the present invention may be any conventional method and is not particularly limited. For example, the cell adhesive substance may be chemically treated with, for example, glutaraldehyde, treated with ozone or irradiated with ultraviolet rays, electron rays or plasma in a specified pattern. Of these methods, irradiation with ultraviolet rays, electrons or plasma through a photo mask bearing the specified pattern is preferred from the viewpoint of introducing crosslinks into the cell adhesive substance in the specified pattern in a short period of time. More preferred is irradiation with ultraviolet rays from the viewpoint of economy and ease of handling.

When irradiation with ultraviolet rays is employed for introducing crosslinks into the cell adhesive substance, the exposure energy (i.e., intensity of ultraviolet rays having a wavelength of 254 nm x irradiation time) which can be employed in the present invention is typically about 100 J/m² to about 10,000 J/m². When the exposure energy is more than about 10,000 J/m², the cells can hardly be detached from the cell culture substrate. On the other hand, when the exposure energy is less than about 100 J/m², the effect of the irradiation with ultraviolet rays can hardly be observed. If the irradiation is carried out with ultraviolet rays having a wavelength other than 254 nm, the exposure energy used is preferably that needed to cause the same amount of crosslinks as when using a wavelength of 254 nm as described above. The irradiation with ultraviolet rays may be repeated as long as the total exposure energy is within the above described range.

The supporting material which can be employed in the present invention is preferably a transparent or translucent material, preferably glass or plastic. Exemplary plastics include polystyrene, polycarbonate, polymethyl methacrylate, acrylic resins, polypropylene, polyethylene, polyester, polyamide, polyvinyl chloride, polyvinylidene fluoride, polyoxymethylene and polytetrafluoroethylene.

The supporting material may be a semipermeable membrane of, for example, regenerated cellulose, cellulose acetate, collagen or chitosan or a porous membrane of, for example, polypropylene, cellulose acetate, polytetrafluoroethylene or polyvinylidene fluoride.

There is no particular limitation with the shape of the supporting material. It can take various shapes such as a dish, plate, film, sheet, bottle, tube, flask and sponge.

The cell culture test material of the present invention comprises an array of coated portions on a supporting material, each of the coated portions comprising a temperature-responsive polymeric compound and, as the cell adhesive substance, collagen or a mixture of collagen as a major component and at least one substance as a minor component selected from the group consisting of fibronectin, laminin, vitronectin, proteoglycan, glycosaminoglycan, thrombospondin, gelatin, lectins, anchorage oligopeptides and adhesive proteins isolated from shellfish, the collagen or the mixture in each of the coated portions having crosslinked parts and non-crosslinked parts in at least one pattern selected from the group consisting of an island-in-sea pattern, a sea-sea pattern and a lamellar pattern and (a) either having the same degree of crosslinking with a regularity of the pattern which differs from the regularity in each of the other coated portions or (b) having a degree of crosslinking which differs from the degree of crosslinking in each of the other coated portions.

The area of each of the coated portions is preferably the same and the number of the coated portions is not particularly limited. Typical numbers of coated portions are 2 to 10 and practically 4 to 8. The shape of the coated portions is typically square, rectangular, triangular, circular or fan-shaped. Preferably each of the coated portions is separated from one another.

Further, the cell culture test material may have two or three groups of coated portions on the same plane, each group typically consisting of two to eight coated portions, with the collagen or the collagen mixture in each of the coated portions in each group having the same degree of crosslinking and the same pattern of crosslinked parts and non-crosslinked parts with a regularity of the pattern which differs from the regularity in each of the other coated portions in the same group, and the pattern in one group which differs from the pattern in another group.

The cell culture test material may also have two or three groups of coated portions on the same plane, each group typically consisting of two to eight coated portions, with the collagen or the collagen mixture in each of the coated portions in one group having crosslinked parts and non-crosslinked parts in the same pattern with a degree of crosslinking which differs from the degree of crosslinking in each of the other coated portions in the same group, and the pattern in one group which differs from the pattern in another group. It is preferred that the area, the number, and the shape of each of the coated portions in each group are the same.

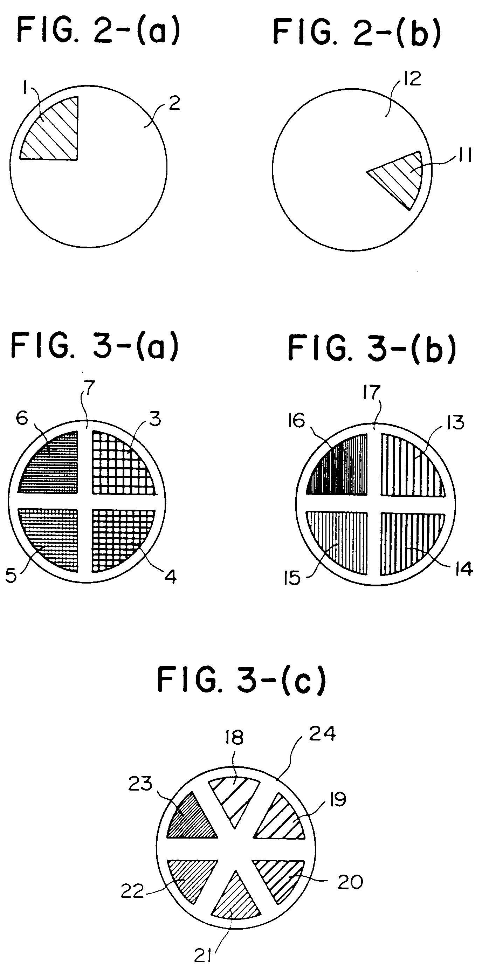

According to one embodiment of the methods of preparing such a cell culture test material, a coating comprising a mixture of the temperature-responsive polymeric compound having an LCST lower than the cell culture temperature and the cell adhesive substance is formed on a circular supporting material, and the coating is sequentially irradiated with ultraviolet rays at different exposure energies through a circular photo mask as shown in FIG. 2-(a) or FIG. 2-(b). The fan-shaped area 1 or 11 allowing the transmission of the ultraviolet rays by its sequential rotation with the photo mask 2 or 12 as a window, respectively, and at least one of the specified three patterns of the present invention such as a gridlike pattern are fixed to the window.

According to another embodiment of the methods, a coating formed on a circular supporting material is irradiated with ultraviolet rays through a circular photo mask as shown in FIG. 3-(a) or FIG. 3-(b) where the four fan-shaped areas 3, 4, 5 and 6 or 13, 14, 15 and 16 allowing the transmission of the ultraviolet rays are provided with the photo mask 7 or 17 as the windows. Four transparencies bearing at least one pattern of the specified patterns with a regularity of the pattern which differs from the regularity in each of the other patterns, are fixed to the windows, respectively. More' specifically, when the pattern of the transparencies is a sea-sea pattern or a lameller pattern, the pattern has a repeat period which differs from the repeat period of the other patterns.

According to a further embodiment of the methods, a coating formed on a circular supporting material is irradiated with ultraviolet rays through a combination of a circular photo mask whose entire surface has been provided with at least one of the specified patterns of the present invention and a circular photo mask having an array of fan-shaped areas as shown in FIG. 3-(c) wherein each of the six fan-shaped areas 18, 19, 20, 21, 22 and 23 in the photo mask 24 allows a different percent transmission of the ultraviolet rays, respectively. More specifically, six transparency areas having percent transmissions of 100% for Area 18; 50% for Area 19; 25 % for Area 20; 12.5% for Area 21; 6.25% for Area 22; and 3.125% for Area 23, respectively, are fixed on the respective windows.

In the above described methods, the coating is formed on the supporting material in the same manner as in the preparation of the cell culture substrate of the present invention, and the introduction of crosslinks into the coating in the specified pattern is most preferably carried out by the irradiation with the ultraviolet rays having a wavelength of 254 nm whose exposure energy is selected from the range of from about 100 $J/m^2$ to about 10,000 $J/m^2$.

By using the cell culture test material, optimal conditions for the attachability, growth and detachability of various types of cells from the cell culture substrate of the present invention can be easily judged.

The examples which follow are given for illustrative purposes and are not meant to limit the invention described herein. The following abbreviations have been used throughout in describing the invention.

PNIPAAm : poly-N-isopropyl acrylamide
DMEM     : Dulbecco's modified Eagle's medium
FBS         : fetal bovine serum

The cell attachability and growth and the cell detachability were calculated from the following equations using the results of eye observation under an inverted phase contrast microscope. The microscopic observation of the cell attachability and growth was conducted at 37°C. For the cell detachability, after the dish had been left to stand on ice for 3 minutes, the microscopic observation was conducted at 20°C.

$$\text{Cell Attachability and Growth (\%)} = \frac{\text{Celle Attached and Grown Area}}{\text{Dish Area}} \times 100$$

$$\text{Cell Detachability (\%)} = \frac{\text{Cell Detached Area}}{\text{Cell Attached and Grown Area}} \times 100$$

Example 1

An aqueous 0.5% (w/v) solution of PNIPAAm having an Mn of $3.5 \times 10^6$ was prepared. The solution was autoclave-sterilized, solubilized again by cooling, and the pH of the solution was adjusted to 3 by addition of HCl. This PNIPAAm solution was mixed with a 0.5% (w/v) type I collagen solution having a pH of 3, solubilized from bovine dermis by pepsinization ("KOKEN CELLGEN I-PC", a product of Koken K.K.) in such a manner that the mixing weight ratio of the collagen to the PNIPAAm became 1:9. 400 $\mu$l of the mixed solution thus prepared was poured onto a commercially available 35 mm dish ("Falcon 3001", a product of Japan Becton) and quickly spread to form a uniform coating on the dish. Then, the coating was dried in a 10°C incubator for 5 hours. As a result, the thickness of the coating was about 2 $\mu$m. The above described procedures were aseptically carried out.

A stainless steel mesh having a size of 500 meshes as shown in FIG. 1-(a) was placed on the coating, and the coating was irradiated with ultraviolet rays having a wavelength of 254 nm using an ultraviolet irradiation apparatus ("XX-100", manufactured by Funakoshi K.K.) with an exposure energy of the ultraviolet ray irradiation of about 10,000 J/m². This irradiation was aseptically carried out.

Then, 2 ml of distilled water of 37°C having a pH of 3 was added to the dish, and the dish was left to stand at 37°C for two hours. Subsequently, the distilled water was removed, and the coating was dyed with Coomassie Brilliant Blue R-250 ( a product of Daiichi Kagaku Chemical Co., Ltd.) at 37°C for one hour. Unreacted dye was washed out with a mixture of acetic acid, methanol and water (volume ratio: 1:1:8), and the coating was dried at 37°C. As a result, it was observed by a microscope that the irradiated portions alone were dyed deep blue in the gridlike pattern of the mesh and the non-irradiated portions were hardly dyed as shown in FIG. 1-(b). This phenomenon shows that crosslinks were introduced into the collagen in an island-in-sea pattern.

Example 2 and Comparative Example 1

The preparation of a coating was carried out in the same manner as in Example 1 except that the exposure energy of the ultraviolet ray irradiation was varied as shown in Table I. As a result, the total area of crosslinked portions was 41%.

The evaluation of cell culture was conducted as follows.

Human dermal fibroblasts were suspended in DMEM containing 10% (v/v) FBS (a product of Gibco) so as to form a final cell density of about $2 \times 10^5$/ml. The cell suspension thus obtained was maintained at 37°C. Separately, a dish having the coating as prepared above was placed on a plate ("Microwarm Plate", a product of Kitazato Supply K.K.) maintained at 37°C and 2 ml of the cell suspension were poured onto the dish. The dish was quickly transferred into a 37°C incubator (air/5 volume % $CO_2$). The cells were cultured for three days.

In Comparative Example 1, the above described procedures were repeated except that the stainless steel mask was not employed for the preparation of the coating.

The results are shown in Table I. Although the cell attachability and growth became slightly poorer when the mask was employed than when the mask was not employed, the cell detachability was dramatically improved. Further, the cells were detached in about one minute when the mask was used compared to about 3 to 5 minutes without the mask. Thus, the rate of cell detachment was also improved.

TABLE I

| Run No. | Exposure Energy of Ultraviolet Ray Irradiation $(J/m^2)$ | Example 2 | | Comparative Example 1 | |
|---|---|---|---|---|---|
| | | Celle Attachability and Growth (%) | Cell Detachability (%) | Cell Attachability and Growth (%) | Cell Detachability (%) |
| 1 | 8000 | 100 | 60 | 100 | 0 |
| 2 | 4000 | 100 | 100 | 100 | 0 |
| 3 | 2000 | 90 | 100 | 100 | 90 |
| 4 | 1000 | 60 | 100 | 80 | 100 |
| 5 | 200 | 10 | 100 | 30 | 100 |

Example 3 and Comparative Example 2

The preparation of a coating was carried out in the same manner as in Example 1 except that a photo mask of quartz glass etched with a lamellar pattern having a line width of 6 μm and a repeat period of 10 μm was employed instead of the mesh. As a result, the total area of crosslinked portions was 41%.

The evaluation of cell culture was carried out in the same manner as in Example 2 except that human chorion fibroblasts were used instead of the human dermal fibroblasts and Eagle's medium was used instead of the DMEM.

In Comparative Example 2, the above described procedures were repeated except that the photo mask was not employed.

The results are shown in Table II. Although the cell attachability and growth became slightly poorer when the photo mask was employed, the cell detachability was dramatically improved and the rate of cell detachment was also improved.

TABLE II

| Run No. | Exposure Energy of Ultraviolet Ray Irradiation $(J/m^2)$ | Example 3 | | Comparative Example 2 | |
|---|---|---|---|---|---|
| | | Cell Attachability and Growth (%) | Cell Detachability (%) | Cell Attachability and Growth (%) | Cell Detachability (%) |
| 1 | 8000 | 100 | 80 | 100 | 0 |
| 2 | 4000 | 30 | 100 | 50 | 100 |

Example 4 and Comparative Example 3

The preparation of a coating was carried out in the same manner as in Example 1 except that the weight mixing ratio of the collagen to the PNIPAAm was 1:19 instead of the 1:9 and a photo mask of quartz glass etched with a sea-sea pattern having a repeat period of 10 μm was employed instead of the mesh. As a result, the total area of crosslinked portions was 30%.

The evaluation of cell culture was carried out in the same manner as in Example 2 except that IMR-90 (fibroblast cell line established from a human fetus lung) was used instead of the human dermal fibroblasts.

In Comparative Example 3, the above described procedures were repeated except the photo mask was not employed.

The results are shown in Table III. As seen in Examples 2 and 3, the cell attachability and growth became slightly poorer when the mask was employed, but the cell detachability was dramatically improved and also the rate of cell detachment was improved.

TABLE III

| Run No. | Exposure Energy of Ultraviolet Ray Irradiation $(J/m^2)$ | Example 4 | | Comparative Example 3 | |
|---|---|---|---|---|---|
| | | Cell Attachability and Growth (%) | Cell Detachability (%) | Cell Attachability and Growth (%) | Cell Detachability (%) |
| 1 | 8000 | 100 | 90 | 100 | 5 |
| 2 | 4000 | 70 | 100 | 80 | 40 |
| 3 | 2000 | 5 | 100 | 15 | 100 |

Example 5 and Comparative Example 4

The same collagen and PNIPAAm as in Example 1 was mixed at a weight mixing ratio of the collagen to the PNIPAAm of 1:9 to give a casting solution. 2.4 ml of the casting solution thus prepared were poured on to a commercially available 90 mm dish ("Falcon 3003", a product of Japan Becton) and quickly spread to form a uniform coating on the dish. The coating was dried in a 10°C incubator for about 10 hours. As a result, the thickness of the coating was about 2 μm. The above described procedures were aseptically carried out.

In order to equally divide the area of the dish into four fan-shaped portions, marks had been put on the rear surface of the dish at every 90°.

Separately, a photo mask having an ultraviolet transmitting window corresponding in size to one of the four equally divided fan-shaped portions of the dish as shown in FIG. 2-(a) was prepared. A mesh having one of the different sizes of meshes as shown in Table IV was fixed to the window of the mask. The photo mask thus prepared was placed on the coating of the dish, and the four fan-shaped portions of the coating were successively irradiated with ultraviolet rays at an exposure energy of the ultraviolet ray irradiation of 2000 $J/m^2$ by using the same ultraviolet irradiation apparatus as in Example 1, changing the mesh and rotating the photo mask at 90°C for each irradiation. These irradiations were aseptically carried out.

The evaluation of cell culture with the dish thus prepared was carried out in the same manner as in Example 2.

In Comparative Example 4, the above described procedures were repeated except that none of the meshes were fixed to the mask.

The results are shown in Table IV. When the mesh was not used, the cell attachability and growth were good, but the cells were hardly detached. On the other hand, when the meshes were used, the cell attachability and growth and the cell detachability varied depending on the regularities of the mesh. As a result, it was found that the mesh in Run No. 1 was most suitable for this cell culture.

TABLE IV

| Example 5 Run No. | Size of Meshes | Line Width (μm) | Aperture Width (μm) | Aperture Ratio (%) | Cell Attachability and Growth (%) | Cell Detachability (%) |
|---|---|---|---|---|---|---|
| 1 | 500 | 25 | 25 | 25 | 100 | 100 |
| 2 | 500 | 18 | 32 | 41 | 100 | 80 |
| 3 | 460 | 30 | 25 | 17 | 80 | 100 |
| 4 | 380 | 40 | 27 | 16 | 90 | 100 |
| Comparative Example 4 Run No. | | | | | | |
| 1 | – | – | – | 100 | 100 | 5 |

Example 6 and Comparative Example 5

The preparation of coating was carried out in the same manner as in Example 5 except that the weight mixing ratio of the collagen to the PNIPAAm was 1:19 instead of the 1:9, a photo mask of quartz glass whose entire surface had been etched with four different regularities of a gridlike pattern as shown in FIG. 3-(a) and Table V was employed, and the exposure energy of ultraviolet ray irradiation of 6000 J/m$^2$ was employed.

The evaluation of cell culture was carried out in the same manner as in Example 4.

In Comparative Example 5, the above described procedures were repeated except that a photo mask having a window which corresponded to one of the four equally divided fan-shaped portions as shown in FIG. 2-(a) was employed instead of the glass photo mask, and the coating of the dish was successively irradiated with the same ultraviolet rays through the photo mask at varied exposure energies as shown in Table VI with the rotation of the mask at 90° for each irradiation.

The results are shown in Table V and Table VI, respectively. Optimal conditions for the cell attachability and growth and the cell detachability could not be found in Comparative Example 5, but for example 6, the cell attachability and growth and the cell detachability varied depending on the pattern of the mask employed. As a result, it was found that Area No. 1 was suitable for this cell culture.

TABLE V

| Area Detachability No. | Line Width (μm) | Aperture (μm) | Aperture Ratio (%) | Cell Attachability and Growth (%) | Cell Detachability (%) |
|---|---|---|---|---|---|
| 1 | 2 | 2 | 25 | 100 | 100 |
| 2 | 5 | 5 | 25 | 95 | 100 |
| 3 | 10 | 10 | 25 | 80 | 100 |
| 4 | 20 | 20 | 25 | 60 | 100 |

TABLE VI

| Area No. | Exposure Energy of Ultraviolet Ray Irradiation (J/m$^2$) | Cell Attachability and growth (%) | Cell Detachability (%) |
|---|---|---|---|
| 1 | 1000 | 5 | 100 |
| 2 | 2000 | 20 | 100 |
| 3 | 4000 | 50 | 95 |
| 4 | 6000 | 100 | 15 |

Example 7 and Comparative Example 6

The preparation of a coating was carried out in the same manner as in Example 5 except that a photo mask of quartz glass etched with four different regularities of a lamellar pattern as shown in FIG. 3-(b) and Table VII was employed and the exposure energy of ultraviolet ray irradiation of 6000 J/m$^2$ was employed.

The evaluation of cell culture was carried out in the same manner as in Example 3.

In Comparative Example 6, the above described procedures were repeated except that a photo mask having a window which corresponded to one of the four equally divided fan-shaped portions as shown in FIG. 2-(a) was employed instead of the glass photo mask and the coating of the dish was succesvively irradiated with the same ultraviolet-rays through the photo mask at varied exposure energies as shown in Table VIII with the rotation of the photo mask at 90° for each irradiation. These irradiations were aseptically carried out.

The results are shown in Table VII and Table VIII, respectively. Optimal conditions for the cell attachability and growth and the cell detachability could not be found in Comparative Example 6, but for example 7, the cell attachability and growth and the cell detachability varied depending on the pattern of the photo mask employed. As a result, it was found that Area No. 2 was suitable for this cell.

TABLE VII

| Area No. | Line Width (μm) | Repeat Period (μm) | Aperture Ratio (%) | Cell Attachability and Growth (%) | Cell Detachability (%) |
|---|---|---|---|---|---|
| 1 | 2 | 4 | 50 | 100 | 90 |
| 2 | 5 | 10 | 50 | 100 | 100 |
| 3 | 10 | 20 | 50 | 70 | 100 |
| 4 | 20 | 40 | 50 | 50 | 100 |

TABLE VIII

| Area No. | Exposure Energy of Ultraviolet Ray Irradiation (J/m$^2$) | Cell Attachability and Growth (%) | Cell Detachability (%) |
|---|---|---|---|
| 1 | 1000 | 5 | 100 |
| 2 | 2000 | 15 | 100 |
| 3 | 4000 | 80 | 40 |
| 4 | 6000 | 100 | 5 |

Example 8 and Comparative Example 7

The preparation of a coating was carried out in the same manner as in Example 5.

In order to equally divide the area of the dish into six fan-shaped portions, marks had been put on the rear surface of the dish at every 60°.

Separately, a photo mask having a window which could transmit ultraviolet rays and which corresponded to one of the six equally divided fan-shaped portions of the dish as shown in FIG. 2-(b) was prepared. A mesh having a size of 500 meshes, a line width of 18 μm, an aperture width of 32 μm and an aperture ratio of 41% was fixed to the window of the photo mask. The photo mask thus prepared was placed on the coating of the dish, and the coating was successively irradiated with ultraviolet rays at varied exposure energies using the same ultraviolet irradiation apparatus as in Example 1 through the photo mask with the rotation of the photo mask at 60° for each irradiation. These irradiations were aseptically carried out.

The evaluation of cell culture with the dish thus prepared was carried out in the same manner as in Example 2.

In Comparative Example 7, the above described procedures were repeated except that the mesh was not used for the window of the photo mask.

The results are shown in Table IX. The cell attachability and growth became slightly poorer when the meshes were employed, but the cell detachability was dramatically improved. The cells were detached in about one minute when the meshes were employed whereas they were detached in about 3 to 5 minutes when the meshes were not employed. Thus, the rate of cell detachment was also improved.

It is clear from Table IX that the optimal exposure energy of ultraviolet ray irradiation was 4000 J/m$^2$, and in this instance, the cell attachability and growth and the cell detachability has further been improved than those in Comparative Example 7.

TABLE IX

| Area No. | Exposure Energy of Ultraviolet Ray Irradiation (J/m$^2$) | Example 8 | | Comparative Example 7 | |
|---|---|---|---|---|---|
| | | Cell Attachability and Growth (%) | Cell Detachability (%) | Cell Attachability and Growth (%) | Cell Detachability (%) |
| 1 | 8000 | 100 | 60 | 100 | 0 |
| 2 | 4000 | 100 | 100 | 100 | 0 |
| 3 | 2000 | 90 | 100 | 100 | 90 |
| 4 | 1000 | 60 | 100 | 80 | 100 |
| 5 | 200 | 10 | 100 | 30 | 100 |
| 6 | 0 | 0 | - | 0 | - |

Example 9 and Comparative Example 8

The preparation of a coating was carried out in the same manner as in Example 8 except that a photo mask (i) having six areas each allowing a different degree of transmittance of the ultraviolet rays as shown in FIG. 3-(c) superimposed with a photo mask (ii) of quartz glass whose entire surface had been etched with a lamellar pattern having a line width of 6 um and a repeat period of 10 μm was employed and the exposure energy of ultraviolet ray irradiation employed was 16000 J/m$^2$. As a result, the total area of crosslinked portions was 40%.

The evaluation of cell culture was carried out in the same manner as in Example 3.

In Comparative Example 8, the above described procedures were repeated except that the photo mask (ii) alone was employed.

The results are shown in Table X. As seen in Example 8, the cell attachability and growth became slightly poorer when the photo mask (i) superimposed with the mask (ii) was employed than when the photo mask (ii) alone was employed, but the cell detachability was dramatically improved and the rate of cell detachment was improved.

It is clear from Table X that the optimal exposure energy of ultraviolet ray irradiation was 8000 J/m², and in this instance, the cell attachability and growth and the cell detachability were further improved than those in Comparative Example 8.

Table X

| Area No. | Exposure Energy Attachability of Ultraviolet Ray Irradiation (J/m²) | Example 9 | | Comparative Example 8 | |
|---|---|---|---|---|---|
| | | Cell Attachability and Growth (%) | Cell Detachability (%) | Cell Attachability and Growth (%) | Cell Detachability (%) |
| 18 | 16000 | 100 | 50 | 100 | 0 |
| 19 | 8000 | 100 | 90 | 100 | 10 |
| 20 | 4000 | 30 | 100 | 50 | 100 |
| 21 | 2000 | 20 | 100 | 30 | 100 |
| 22 | 1000 | 20 | 100 | 30 | 100 |
| 23 | 500 | 0 | - | 0 | - |

Example 10 and Comparative Example 9

The preparation of a coating was carried out in the same manner as in Example 8 except that the weight mixing ratio of the collagen to the PNIPAAm was 1:39 instead of the 1:9 and a photo mask (i) having a window as shown in FIG. 2-(b) superimposed with a photo mask (ii) of quartz glass whose entire surface had been etched with a sea-sea pattern having a repeat period of 10 μm was employed, and the exposure energy of ultraviolet ray irradiation was varied as shown in Table XI. As a result, the total area of crosslinked portions was 30%.

The evaluation of cell culture was carried out in the same manner as in Example 4.

In Comparative Example 9, the above described procedures were repeated except that the photo mask (i) alone was employed.

The results are shown in Table XI. As seen in Examples 8 and 9, the cell attachability and growth became slightly poorer when the photo mask (ii) was employed than when the photo mask (ii) was not employed, but the cell detachability was dramatically improved and the rate of cell detachment was improved.

It is clear from Table XI that the optimal exposure energy of ultraviolet ray irradiation was 8000 J/m² and in this instance, the cell attachability and growth and the cell detachability was further improved than those in Comparative Example 9.

TABLE XI

| Area No. | Exposure Energy of Ultraviolet Ray Irradiation $(J/m^2)$ | Example 10 | | Comparative Example 9 | |
|---|---|---|---|---|---|
| | | Cell Attachability and growth (%) | Cell Detachability (%) | Cell Detachability and Growth (%) | Cell Detachability (%) |
| 1 | 16000 | 100 | 40 | 100 | 0 |
| 2 | 8000 | 100 | 100 | 100 | 60 |
| 3 | 4000 | 20 | 100 | 40 | 100 |
| 4 | 2000 | 0 | - | 0 | - |
| 5 | 1000 | 0 | - | 0 | - |
| 6 | 500 | 0 | - | 0 | - |

**Claims**

1. A cell culture substrate having a cell growth area comprising (a) a temperature-responsive polymeric compound having an LCST lower than the culture temperature and (b) a cell adhesive substance capable of adhering the cell to be cultured without any degeneration of the cell coated on a supporting material, said cell adhesive substance comprising collagen or a mixture of collagen as a major component and at least one substance as a minor component selected from the group consisting of fibronectin, laminin, vitronectin, proteoglycan, glycosaminoglycan, thrombospondin, gelatin, lectins, anchorage oligopeptides and adhesive proteins isolated from shellfish and having crosslinked parts and non-crosslinked parts in at least one pattern selected from the group consisting of an island-in-sea pattern, a sea-sea pattern and a lamellar pattern.

2. The cell culture substrate of Claim 1, wherein said temperature-responsive polymeric compound is selected from the group consisting of a poly-N-substrated acrylamide derivative or copolymer thereof, a poly-N-substituted methacrylamide derivative or copolymer thereof, polyvinylmethylether, and a partially acetylated polyvinylalcohol.

3. The cell culture substrate of Claim 2, wherein said temperature-responsive polymeric compound is a poly-N-substituted acrylamide derivative.

4. The cell culture substrate of Claim 3, wherein said poly-N-substituted acrylamide derivative is poly-N-isopropyl acrylamide.

5. The cell culture substrate of Claim 1, wherein the weight ratio of said temperature-responsive polymeric compound to said cell adhesive substance is about 49:1 to about 9:1.

6. The cell culture substrate of Claim 1, wherein the thickness of the coating comprising said temperature-responsive polymeric compound (a) and said cell adhesive substance (b) is at least 0.5 µm.

7. The cell culture substrate of Claim 1, wherein said temperature-responsive polymeric compound and said cell adhesive substance are in the form of at least one coating on said supporting material, said coating being either
   (a) a mixture of said polymeric compound and said cell adhesive substance,
   (b) a sequential layer wherein a first layer comprises said polymeric compound and a second layer comprises said cell adhesive substance or
   (c) a sequential layer wherein a first layer comprises said cell adhesive substance and a second layer comprises said polymeric compound.

8. The cell culture substrate of Claim 1, wherein the total area of said crosslinked parts in the cell adhesive substance is not greater than 50% of the cell growth area.

9. The cell culture substrate of Claim 8, wherein said total area is not greater than 30% of the cell growth area.

10. The cell culture substrate of Claim 1, wherein said pattern is an island-in-sea pattern and the size of islands and the distance between two adjacent islands are not greater than the size of the cell to be cultured.

11. The cell culture substrate of Claim 10, wherein said size of islands and said distance between two adjacent islands are 0.1 μm to 50 μm.

12. The cell culture substrate of Claim 1, wherein said pattern is a sea-sea pattern or a lamellar pattern and the repeat period is not greater than twice the size of the cell.

13. The cell culture substrate of Claim 12, wherein said repeat period is not greater than 100 μm.

14. The cell culture substrate of Claim 13, wherein said repeat period is not greater than 40 μm.

15. A method of preparing a cell culture substrate which comprises forming a coating comprising
    (a) a temperature-responsive polymeric compound having an LCST lower than the cell culture temperature and
    (b) collagen or a mixture of collagen as a major component and at least one substance as a minor component selected from the group consisting of fibronectin, vitronectin, laminin, proteglycan, glycosaminoglycan, thrombospodin, gelatin, lectins, anchorage oligopeptides and adhesive proteins isolated from shellfish,
    at a weight ratio of about 49:1 to about 9:1, said coating being formed to a thickness of at least about 0.5 μm on a supporting material, and introducing crosslinks into said collagen or said mixture in said coating in at least one pattern selected from the group consisting of an island-in-sea pattern, a sea-sea pattern and a lamellar pattern.

16. The method of Claim 15, wherein the introduction of crosslinks into said collagen or said mixture is carried out by irradiating said coating with ultraviolet rays having a wavelength of 254 nm at an exposure energy of about 100 to 10,000 J/m$^2$ through a photo mask bearing said pattern allowing the transmission of said ultraviolet rays.

17. A cell culture test material which comprises an array of coated portions on a supporting material, each of said coated portions comprising:
    i) a temperature-responsive polymeric compound and,
    ii) collagen or a mixture of collagen as a major component and at least one substance as a minor component selected from the group consisting of fibronectin, laminin, vitronectin, proteoglycan, glycosaminoglycan, thrombospondin, gelatin, lectins, anchorage oligopeptides and adhesive proteins isolated from shellfish,
    each of said coated portions having substantially the same area, said collagen or said mixture in each of said coated portions having crosslinked parts and non-crosslinked parts in at least one pattern selected from the group consisting of an island-in-sea pattern, a sea-sea pattern and a lamellar pattern and the same degree of crosslinking, and the regularity of said pattern which differs from the regularity in each of the other coated portions.

18. A cell culture test material which comprises an array of coated portions on a supporting material, each of said coated portions comprising:
    i) a temperature-responsive polymeric compound and,
    ii) collagen or a mixture of collagen as a major component and at least one substance as a minor component selected from the group consisting of fibronectin, laminin, vitronectin, proteoglycan, glycosaminoglycan, thrombospondin, gelatin, lectins, anchorage oligopeptides and adhesive proteins isolated from shellfish,
    each of said coated portions having substantially the same area, and said collagen or said mixture in each of said coated portions having crosslinked parts and non-crosslinked parts in at least one pattern selected from the group consisting of an island-in-sea pattern, a sea-sea pattern and a lamellar pattern with a degree of crosslinking which differs from the degree of crosslinking in each of the other coated portions.

19. The cell culture test material of Claim 17 or Claim 18, wherein each of said coated portions is detached from one another.

20. The cell culture test material of Claim 17 or Claim 18, wherein the number of said coated portions is 2 to 10.

21. The cell culture test material of Claim 17, wherein said coated portions are divided into two or three groups with each group consisting of a plurality of said coated portions and said collagen or said mixture in each of said coated portions has crosslinked parts and non-crosslinked parts in the same pattern in each group with the same degree of crosslinking and a regularity of the pattern which differs from the regularity in each of the other coated portions in the same group and said pattern in one group which differs from the pattern in another group.

22. The cell culture test material of Claim 21, wherein each group has the same number of said coated portions.

23. The cell culture test material of Claim 18, wherein said coated portions are divided into two or three groups with each group consisting of a plurality of said coated portions and said collagen or said mixture in each of said portions has crosslinked parts and non-crosslinked parts in the same pattern in each group with a degree of crosslinking which differs from the degree of crosslinking in each of the other coated portions in the same group and said pattern in one group which differs from the pattern in another group.

24. The cell culture test material of Claim 23, wherein each group has the same number of said coated portions.

# FIG. 1-(a)

# FIG. 1-(b)

FIG. 2-(a)

FIG. 2-(b)

FIG. 3-(a)

FIG. 3-(b)

FIG. 3-(c)

EP 0 529 751 A1

European Patent Office

**EUROPEAN SEARCH REPORT**

Application Number

EP 92 25 0209

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 373 626 (MITSUBISHI KASEI CORPORATION)<br>* the whole document * | 1-4,8-14 | C12N5/00 |
| Y | EP-A-0 387 975 (W.R. GRACE & CO. - CONN.)<br>* claims * | 1-4,8-14 | |
| A | EP-A-0 402 718 (KANEGAFUCHI KAGAKU KOGYO KABUSHIKI KAISHA)<br>* abstract; claims; figure 1 * | 1,8-24 | |
| A | EP-A-0 382 214 (KAO CORPORATION)<br><br>* the whole document * | 1-4,<br>15-16 | |
| A | EP-A-0 246 013 (KOKEN CO. LTD.)<br><br>* the whole document * | 8-9,<br>15-24 | |
| | ----- | | **TECHNICAL FIELDS SEARCHED (Int. Cl.5)**<br><br>C12N |

The present search report has been drawn up for all claims

| Place of search | Date of completion of the search | Examiner |
|---|---|---|
| THE HAGUE | 29 DECEMBER 1992 | BEVAN S.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

22